# EUROPÄISCHE PATENTSCHRIFT

(11) **EP 3 328 470 B1**
(45) Veröffentlichungstag und Bekanntmachung des Hinweises auf die Patenterteilung: **19.06.2019**
(21) Anmeldenummer: 16756595.1
(22) Anmeldetag: 28.07.2016
(51) Int. Cl.: A61M 5/24, A61M 5/315, A61M 5/46, A61M 5/20, A61M 5/32, A61M 5/28

(54) **SPRITZE**
SYRINGE
SERINGUE

(30) Priorität: 30.07.2015 DE 102015112564
(43) Veröffentlichungstag der Anmeldung: 06.06.2018
(73) Patentinhaber: F+K Innovationen GmbH & Co.KG, 76534 Baden-Baden (DE)
(72) Erfinder: FUCHS, Karl-Heinz, 78315 Radolfzell (DE)
(74) Vertreter: Patentanwälte und Rechtsanwalt Weiß, Arat & Partner mbB
(86) Internationale Anmeldenummer: PCT/EP2016/068088
(87) Internationale Veröffentlichungsnummer: WO 2017/017229

(56) Entgegenhaltungen:
- WO-A1-2013/064676
- US-A1- 2014 005 609

## Beschreibung

### Technisches Gebiet

Die Erfindung betrifft eine Spritze nach dem Oberbegriff des Anspruches 1.

### Stand der Technik

Aus dem Stand der Technik sind verschiedene Einmalspritzen bekannt, welche aber sehr materialaufwändig sind und entsprechend viel Müll verursachen. Die bekannten Einmalspritzen werden nach dem Gebrauch als Ganzes weggeworfen und sind grundsätzlich aber noch verwendbar, d.h. die Spritzen können nochmals aufgezogen werden, was wiederum den Nachteil birgt, dass sie im Drogenmilieu missbräuchlich eingesetzt werden, was gerade die Ansteckungsgefahr von über die Blutbahn übertragbare Krankheiten erhöht.

In diesem Zusammenhang wird auf die WO 2013/064676 A1 verwiesen. Dort ist eine Einmalspritze zur Einbringung eines Mediums in den menschlichen Körper mit einem Lagerelement oder einer Ampulle und einem Ausbringelement, wobei das Lagerelement oder die Ampulle lösbar mit dem Ausbringelement verbunden ist. Weiter wird auf die US 2014/0005609 A1 verwiesen, welche einen federgelagerten Nadelschutz aufweist, welcher bei Aufdrücken auf eine Haut nachgibt und dabei die Spritze aus dem Nadelschutz austritt.

### Aufgabe der Erfindung

Aufgabe der Erfindung ist es, eine Einmalspritze zur Verfügung zu stellen, welche die Nachteile aus dem Stand der Technik weitgehend abstellt und einen benutzerfreundlichen, verletzungsarmen Aufbau bei einer System unabhängigen Anwendung aufweist.

### Lösung der Aufgabe

Die Aufgabe wird durch die Merkmale des Anspruches 1 gelöst.

Bei der erfindungsgemässen Spritze handelt es sich um eine Spritzenkonstruktion, welche alle über eine Spritze einbringbare Medien in den menschlichen Körper einbringen kann. In einem erfindungsgemässen Ausführungsbeispiel handelt es sich um eine Einmalspritze, wobei nicht die gesamte Konstruktion einmalig nutzbar ist. Ein Ausbringelement ist derart ausgeführt, dass es wiederverwendbar ist. Das Ausbringelement dient zur Nutzung eines Spritzenkörpers, der durch das Ausbringelement betätigt werden kann. Nach der einmaligen Betätigung ist der Spritzenkörper zur Entsorgung vorgesehen.

Mit der WO 2013/064676 wurde ein System angemeldet, welches den Anforderungen der Nachhaltigkeit und dem Missbrauch im Drogenmilieu sowie der Materialverschwendung genügt. Die neue Anmeldung setzt auf dieser Anmeldung auf und soll die Verletzungsgefahr durch die Nadel eliminieren sowie eine bedingte Benutzerunabhängigkeit und Automation garantieren.

Die erfindungsgemässe Spritze besteht aus dem Ausbringelement und dem Lagerelement. Das Ausbringelement ist ein im Wesentlichen einstückig aus Kunststoff hergestelltes Handinstrument.

Das Ausbringelement besteht in erster Linie aus einer Ausbringhülse und einem Blattfedersystem. Das Blattfedersystem besteht aus einem Blattfederboden und einem Deckenbereich. Der Deckenbereich weist eine Ausbringhülse auf, wobei sich die Ausbringhülse von dem Deckenbereich andernseits der dem Blattfederboden zugewandten Seite weg erstreckt.

Ausserdem umfasst der Blattfederboden einen Kolben, wobei der Kolben sich von dem Blattfederboden hin zu dem Deckenbereich erstreckt und in die Ausbringhülse hinein verschiebbar ist. Die Verschiebbarkeit des Kolbens in die Ausbringhülse wird dadurch erreicht, dass zwischen dem Blattfederboden und dem Deckenbereich ein Kompressionsfederelement einstückig mit dem Blattfederboden und dem Deckenbereich ausgebildet ist. Bei dem Kompressionsfederelement handelt es sich um zusammendrückbare Federelemente, welche einstückig mit dem dem Deckenbereich und dem Blattfederboden verbunden sind. Die Anzahl der Kompressionsfederelemente ist vom Einzalfall abhängig. In einem bevorzugten Ausführungsbeispiel erstrecken sich jeweils drei bis vier Kompressionsfederelemente einends und andernends von dem Blattfederboden zu dem Deckenbereich, so dass eine geschlossene Konstruktion entsteht, in der mittig der Kolben vom Blattfederboden zu dem Deckenbereich erstreckt.

Das Blattfedersystem umfasst einen Kolben und eine um den Kolben herum angeordnete Spiralfeder, wobei der Kolben und die Spiralfeder in die Ausbringhülse einschiebbar ist und die Spiralfeder zwischen dem Blattfederboden und einem Spiralfederanschlag des Lagerelements angeordnet ist, wobei der Kolben an den Stopfen anschlägt, die Spiralfeder aber an den Spiralfederanschlag. Der Spiralfederanschlag ist Teil des Lagerelements. Beim Einschieben des Lagerelements in die Ausbringhülse wird die Spiralfeder zusammengedrückt. Damit das Kompressionsfederelement nicht ungewollt auseinander gedrückt wird, umfasst das Kompressionsfederelement ein Rückhalteelement. Dabei handelt es sich beispielsweise um eine Folie, welche derart in dem Kompressionsfederelement angeordnet ist, dass beim Einführen des Lagerelements in die Ausbringhülse das Kompressionsfederelement nur in eine definierte Position ausdehnbar ist. Der Stopfen ist in dem Lagerelement verschiebbar und abgedichtet.
Das Lagerelement weist einen Spritzenkörper auf. In dem Spritzenkörper ist ein Wirkstoff in flüssigem Aggregatzustand eingefüllt. Der Spritzenkörper weist einends eine Injektionsnadel und andernends einen Einrastflansch auf, Daneben ist in dem Spritzenkörper ein bewegbarer Stopfen angeordnet, welcher bei Betätigung durch den Kolben den Wirkstoff aus der Injektionsnadel drückt.

Die Ausbringhülse weist einen Führungskanal und eine Einstichanschlagsfläche auf, wobei die Einstichanschlagsfläche andernends des Deckenbereichs angeordnet ist und eine Aufnahme für einen Faltenbalg umfasst. Der Faltenbalg ist mit der Aufnahme verbunden oder einstückig ausgebildet. Der Faltenbalg legt sich während der Anwendung der erfindungsgemässen Spritze um die Injektionsnadel. Die Anwendung erstreckt sich hierbei von dem Einführen des Lagerelements in die Ausbringhülse über das Einstechen der Injektionsnadel in die Haut des Patienten und die Abgabe des Wirkstoffs bis hin zur Freigabe des Lagerelements aus dem Ausbringelement. Vorteilhaft an dem Faltenbalg ist, dass es während der gesamten Anwendung einen Verletzungsschutz bietet. Vorteilhaft an der Aufnahme ist, dass es neben der Aufnahme des Faltenbalgs auch die Ausbringhülse zusätzlich stabilisiert.

Erfindungsgemäss weist das Ausbringelement eine erste Verriegelung auf. Die erste Verriegelung dient der Verriegelung und dient der Festlegung des Lagerelements in dem Ausbringelement in einer ersten Position. Dabei gleitet der Einrastflansch des Lagerelements in einem Führungskanal derart entlang bis es selbständig durch das erste Verriegelungselement in der ersten Position verriegelt ist. Dabei kann das erste Verriegelungselement Gleitflächen aufweisen, welche das Entlanggleiten an dem Verriegelungselement erlauben, aber ein Zurückgleiten in Richtung der Aufnahme verhindern. In der ersten Position schlägt der Kolben in einer definierten Position mit entsprechend definierter Stauchung der Spiralfeder an dem Stopfen des Spritzenkörpers an.

Nach der manuellen Entriegelung gleitet das Lagelement in der Ausbringhülse proximal hin zu dem zu behandelnden Körper und wird von dem zweiten Verriegelungselement festgelegt. Dabei kann das zweite Verriegelungselement Gleitflächen aufweisen, welche das Entlanggleiten und Passieren an dem zweiten Verriegelungselement erlauben, aber ein Zurückgleiten in Richtung der Aufnahme verhindern.

Beim Festlegen des Lagerelements an der zweiten Verriegelung wird die zweite Position erreicht. In der zweiten Position ragt die Injektionsnadel in gewünschter Länge aus der Ausbringhülse hinaus. Dabei kann sie entweder komplett oder auch nur teilweise von dem Faltenbalg umfangen sein. Das Gleiten des Lagerelements von der ersten Position in die zweite Position in Richtung der Einstichanschlagsfläche wird durch die angespannte Spiralfeder erreicht, wobei sich die Spiralfeder tielweise entspannt. In der zweiten Position kann der Nutzer nun durch Zusammendrücken des Kompressionsfederelements den Kolben betätigen, welcher den Wirkstoff aus dem Spritzenkörper drückt. Nach der Anwendung kann der Nutzer die zweite Verriegelung lösen und die Spiralfeder drückt das benutzte Lagerelement automatisch aus der Ausbringhülse, sodass der Nutzer das Lagerelement vorteilhaft und einfach entsorgen kann.

Der Führungskanal erstreckt sich im Wesentlichen über die gesamte Länge der Ausbringhülse. Der Führungskanal kann dabei derart ausgebildet sein, dass die Ausbringhülse eine geschlossene Röhrenkonstruktion ist oder seitlich geöffnet beziehungsweise geschlitzt ist.

Die Ausbringhülse kann auch einen weiteren Führungskanal aufweisen, wobei auch ein weiterer Einrastflansch mit dem weiteren Führungskanal wirkverbindbar ist. Das bedeutet, dass beispielsweise der weitere Führungskanal 180° zu dem ersten Führungskanal angeordnet ist und das Lagerelement einen weiteren Einrastflansch aufweist, welcher 180° zu dem ersten Einrastflansch angeordnet ist.
Dabei kann vorgesehen sein, dass die erste Verriegelung den Einrastflansch verriegelt und löst und die zweite Verriegelung den weiteren Eintrastflansch verriegelt und löst.

Weiter kann die erste Verriegelung ein erstes Federelement umfassen, wobei das erste Federelement einstückig mit dem Blattfedersystem verbunden ist. Dadurch wird vorteilhaft erreicht, dass die erste Verriegelung beim Vorbeigleiten des Einrastflansches nachgibt und beim Lösen das gleiche Federelement dem Nutzer eine einfach Nutzung erlaubt.

In gleicher Weise kann die zweite Verriegelung ein zweites Federelement umfassen, wobei das zweite Federelement ebenfalls einstückig mit dem Blattfedersystem verbunden ist.

Die Einmalspritze ist im Wesentlichen zweiteilig aufgebaut, d.h. es ist ein Lagerelement als Spritzenkörper und ein Ausbringelement als Adapter vorhanden.

Die beiden Teile sind trennbar miteinander gekoppelt, wobei die Koppelung derart gestaltet ist, dass das entleerte Lagerelement vom Ausbringelement abnehmbar ist und das Ausbringelement wieder verwendbar mit einem gefüllten Lagerelement wieder zur Anwendung kommen kann.

Der Inhalt des Lagerelementes spielt in diesem Zusammenhang eine untergeordnete Rolle.

Es ist auch denkbar, dass das Lagerelement und der Spritzenkörper aus verschiedenen Materialien wie Kunststoff oder Glas hergestellt ist.

### Handhabung des Systems

Beim Bestücken des Ausbringelementes mit dem Lagerelement wird das Injektionssystem über eine Spiralfeder vorgespannt. Durch das Aufsetzen des Injektionssystems auf dem gewünschten Hautbereich wird der Einstichweg durch den Einstichanschlag begrenzt. Das Handling des Systems kann in der Selbstmedikation oder über den Arzt ausgeführt werden, da die Griffflächen für beide Varianten integriert wurden. Durch das horizontale Verschieben und dadurch Entriegeln des Systems durch den Anwender gibt das vertikale Federelement den Spritzenkörper frei, so dass er sich in seiner Position durch die vorgespannte Spiralfeder verschiebt und den Einstich in die Haut generiert. Der Anwender wird im Folgeschritt das Injektionssystem über die Griffflächen betätigen und dadurch die Injektion auslösen. Nach der Injektion wird die Nadel mit dem Spritzenkörper durch den Auswerfer aus dem System entsorgt.

Bei dem Lagerelement handelt es sich um einen nicht nachfüllbaren Einmalbehälter, welcher nur zerstörend wieder aufegfüllt werden kann.

Das Lagerelement oder der Spritzenkörper besteht aus dem Glas- oder Kunststoffkörper mit einem beweglichen Stopfen ohne Betätigungskolben am distalen Ende und einer Injektionsnadel an dem proximalen Ende, welche mit einer Schutzkappe vor Verletzungen geschützt wird.

Um die Verletzungsgefahr beim manuellen Bestücken des Ausbringelementes zu reduzieren, wird das Lagerelement über die Injektionsschutzkappe bis zum Einrasten in das Ausbringelement gedrückt. In diesem Zustand liegt die Injektionsnadel verletzungsfrei innerhalb des Ausbringelementes und des Einstichanschlages.

Nach dem Abziehen der Schutzkappe und dem manuellen Auslösen des Injektionsvorganges durch die vorgespannte Feder wird das Lagerelement in seiner Position verschoben, so dass die Injektionsnadel bis zum Einstichanschlag stechen kann und die Injektion durch das Betätigen des Kolbens über die beiden Kunststoffblattfedern ausgeführt werden kann.

Das Ausführungsbeispiel der erfindungsgemäßen Einmalspritze ist derart angeordnet, dass der Kolben des Ausbringelementes mit dem Stopfen des Lagerelementes, Spritzenkörper zusammenwirkt, dabei greift der Kolben in das Lagerelement und bewegt den Stopfen in Richtung der Nadel. Der Kolben ist einstückig mit den beiden Federelementen ausgebildet, wobei die Federelemente den Kolben durch seine Anbindung am Ausbringelement rückgestellt wird.

Eine weitere Besonderheit des Ausbringelementes ist die Spiralfeder um den Kolben, welche beim Einschieben des Spritzenkörpers bzw. des Lagerelementes um den Einstichweg vorgespannt wird. Beim manuellen Auslösen wird der Haltenocken horizontal gegen ein Federelement verschoben, so dass der Spritzenkörper bis zum Entnahmenocken verschoben wird. Nach der Injektion wird der Entnahmenocken ebenfalls manuell horizontal bewegt, so dass das Lagerelement aus dem Ausbringelement über die Schutzkappe entnommen werden kann.

Eine weitere Besonderheit des Ausbringelementes besteht darin, dass alle beiden Ver- und Entriegelungsnocken und manuell zu bewegenden Laschen sowie das Kunststofffederelement mit Kolben und Lagerelementhalterung einstückig als Kunststoffteil geformt sind.

Die erfindungsgemäße Einmalspritze weist drei bzw. vier Griffflächen als Betätigungsflächen aus, welche jeweils endseitig der Kraftausdehnungsrichtung des Federelementes angeordnet sind und die Handhabung für den Arzt oder in der Selbstmedikation zulässt und ebenfalls einstückig mit dem Federelement ausgebildet sind.

Dadurch wird vorteilhaft erreicht, dass der Kolben durch drei an den Betätigungsflächen anliegenden Fingern, welche gegen die Kraft des Federelementes zueinander gedrückt werden, in das Lagerelement hineingestoßen wird. Beim Lösen des Fingergriffes stellt das Federelement die Betätigungsflächen in die Ausgangsposition zurück.

Die Benutzerunabhängigkeit wird durch den automatisierten Ablauf der Injektion in Verbindung mit dem manuellen Auslösen und Entriegeln bei hoher Sicherheit und Reduktion durch Verletzungen der Injektionsnadel erreicht.

Das Ausbringelement weist in einem Ausführungsbeispiel eine Entriegelungsvariante auf. Dabei ist ein Entriegelungselement in einem oberen Bereich an dem Griff angebracht. Der obere Bereich ist der Bereich des Griffs, welcher zu dem Spritzenkörper hin ausgerichtet ist. Eine Vertikalentriegelung ist mit dem Federelement im Bereich der Grifffläche verbunden. Dabei handelt es sich um die erste manuelle Entriegelung des Systems zur Injektion.

Die zweite Entriegelung stellt das Entriegelungselement dar. Das Entriegelungselement gibt nach Betätigung die Spritze aus dem System frei. Das Blattfederelement bringt den Kolben in Position, damit die Spiralfeder das Lagerelement mit dem Spritzenkörper bewegen kann.

Das Entriegelungselement wird über die manuell gesteuerte Grifffläche und dem Federelement betätigt.

### Figurenbeschreibung

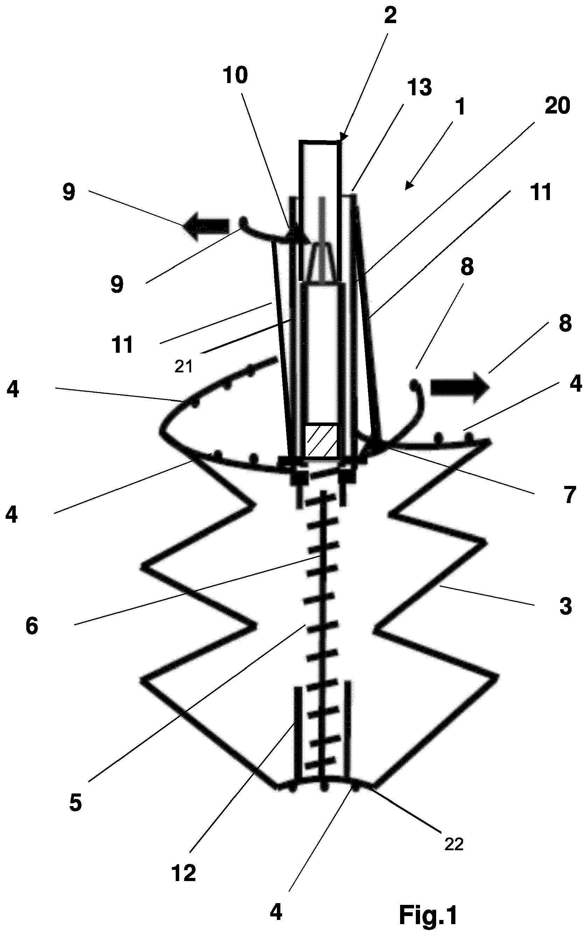

Weitere Vorteile, Merkmale und Einzelheiten der Erfindung ergeben sich aus der nachfolgenden Beschreibung bevorzugter Ausführungsbeispiele sowie anhand der Zeichnungen, diese zeigen in
Fig. 1 zeigt einen Querschnitt einer erfindungsgemäßen Einmalspritze 1 bestehend aus dem Ausbringelement 20 mit einem bestückten Lagerelement 2. Außerdem ist eine erste Verriegelung 7 und eine zweite Verriegelung 9 gezeigt. Die vorgespannte Spiralfeder 5, welche zentrisch auf dem Kolben 6 mit der Blattfeder 3 mit den integrierten Griffflächen 4 einstückig verbunden ist, zeigt das System vor der Injektion. Ausserdem ist eine Federführung 12 gezeigt.
   Eine Federführung 12 ragt zylindrisch von einem Blattfederboden 22 ab und dient zur teilweisen Aufnahme der Spiralfeder 5, sowie des Kolbens 6. Einends ist die Spiralfeder 5 über die Federführung 12 mit der Grifffläche 4 einstückig verbunden. Der Kolben 6 wiederum ist ebenfalls mit der Grifffläche 4 verbunden. Die Spiralfeder 5 umgrift dabei den Kolben 6. Durch das Zusammendrücken der Blattfeder 3 aktiviert der Kolben 6 das Lagerelement 2, so dass ein Stopfen 18 den Inhalt des Lagerelements 2 über die Injektionsnadel 16 ausbringt, wobei die Injektionsnadel 16 an einem ersten Ende des Spritzenkörpers 17 umfasst ist und an einem zweiten Ende der Stopfen 18 und ein Einrastflansch19 vorhanden sind.
   Ausserdem ist die erste Verriegelung 7 und ein Federelement 11 zu erkennen, welche als ein Teil des Ausbringelements 20 ausgebildet sind.
   Zuletzt ist in der Figur 1 ein Einstichanschlagsfläche 13 zu erkennen, welche bei der Injektion an die Haut anliegt.

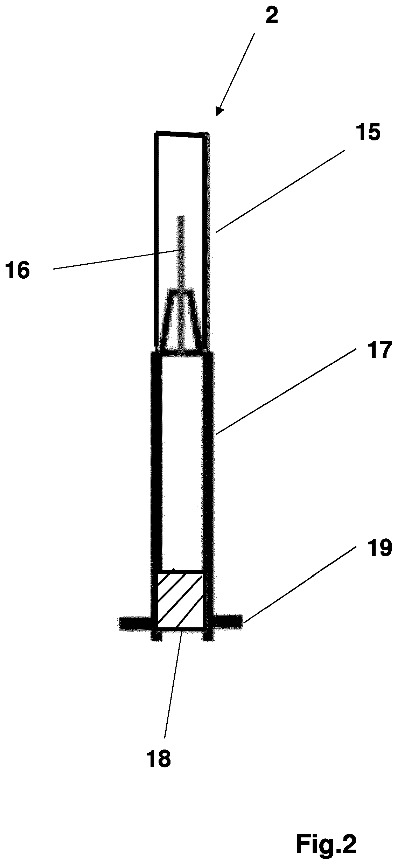

Figur 2 zeigt das Lagerelement 2 als Querschnitt mit einer Schutzkappe 15 über der Injektionsnadel 16 auf dem Spritzenkörper 17 sowie den Stopfen 18 als beweglicher Boden. Der Einrastflansch 19 hat die Aufgabe, das System im Ausbringelement 20 zu fixieren.

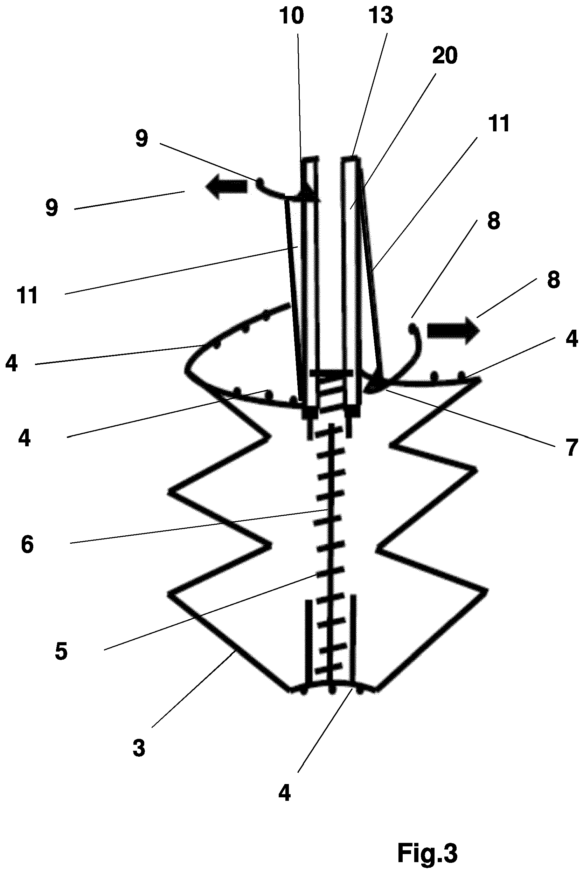

Figur 3 zeigt einen Querschnitt durch das Ausbringelement 20 ohne das Lagerelement 2. Im oberen Bereich sitzt die zweite Verriegelung 9 auf dem Federelement 11, im Bereich der Grifffläche 4 sitzt das Entriegelungselement 8 verbunden mit dem Federelement 11. Die Griffflächen 4 geben das Handling für die Selbstmedikation oder die Anwendung vom Arzt vor. Das doppelte Blattfederelement 3 bringt den Kolben 6 in Position. Am oberen Ende des Systems ist die Einstichanschlagfläche 13 verwirklicht. Am oberen Ende bedeutet hierbei, dass es sich um das dem Blattfederboden 22 entgegen liegende Ende der des Ausbringelements 20. Die Einstichanschlagfläche 13 ist zylindrisch an das Blattfederelement 3 einstückig angeformt.

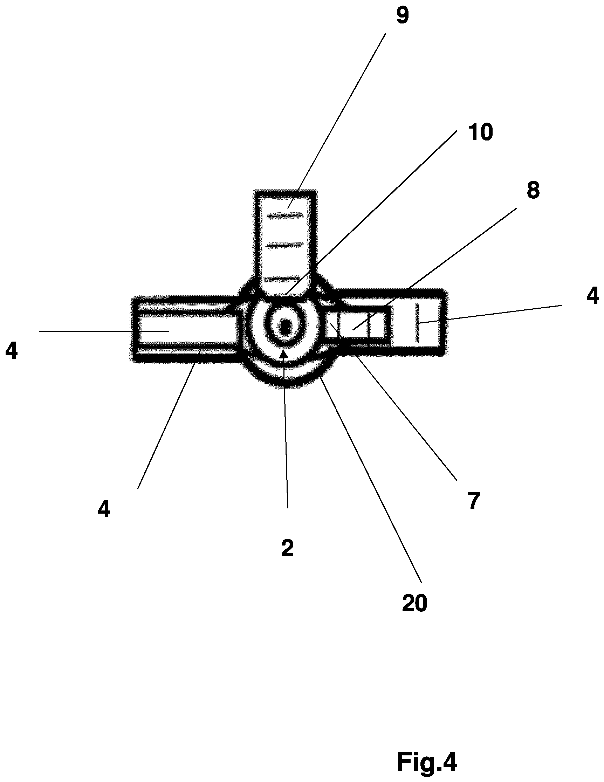

Figur 4 zeigt das System in der Draufsicht mit den Griffflächen 4, die erste Verriegelung 7 mit einem Verriegelungsnocken 8 sowie die zweite Verriegelung 9 mit einem weiteren Verriegelungsnocken 10.

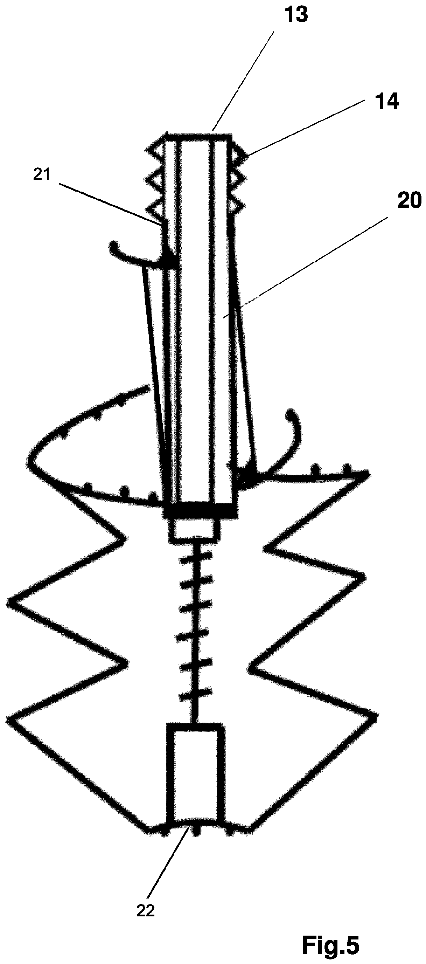

Figur 5 zeigt eine Variante des Systems mit einem Faltenbalg 14, integriert am Ausbringelement 20 zum Schutz der Nadel 16 im Lagerelement 2.

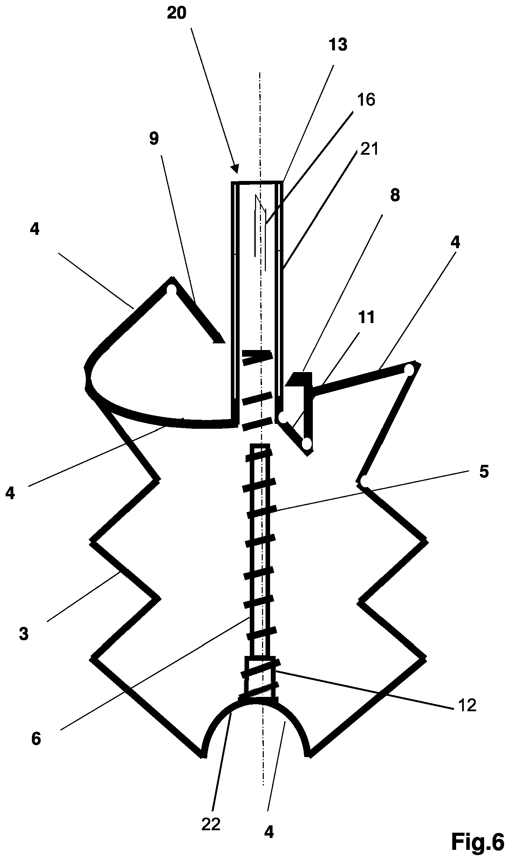

Figur 6 zeigt einen Querschnitt durch das Ausbringelement 20 mit einer Entriegelungsvariante. Im oberen Bereich sitzt das Entriegelungselement 9 mit der Griffvariante für den Arzt und für die Selbstmedikation ausgelegt. Die Vertikalentriegelung 8 verbunden mit dem Federelement 11 im Bereich der Grifffläche 4 ist die erste manuelle Entriegelung des Systems zur Injektion. Die zweite Entriegelung über das Entriegelungselement 9 gibt die Spritze aus dem System frei. Das Blattfederelement 3 bringt den Kolben 6 in Position, damit die Spiralfeder 5 das Lagerelement 2 mit dem Spritzenkörper 17 bewegen kann.

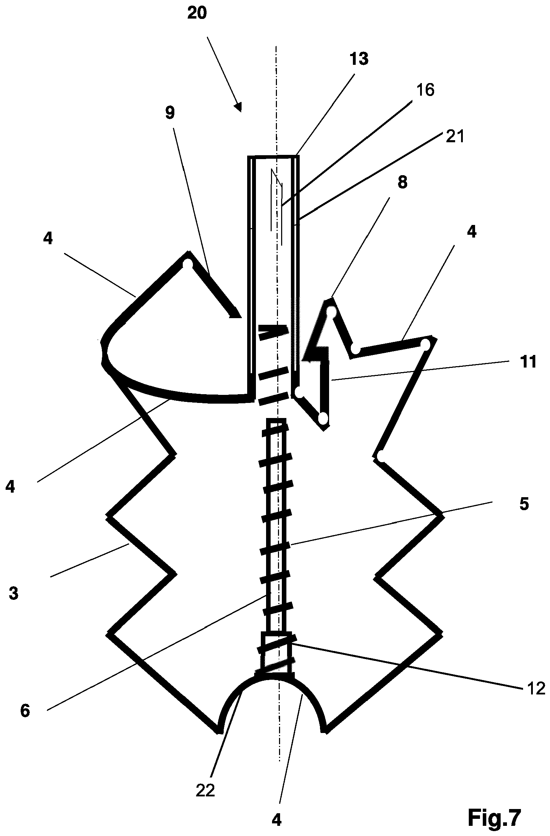

Fig.7 zeigt eine Variante der vertikalen Entriegelung 8 als Querschnitt durch das Ausbringelement 20. Das Entriegelungselement 8 wird über die manuell gesteuerte Grifffläche 4 und dem Federelement 11 betätigt.

### Bezugszeichenliste

1 Spritze
2 Lagerelement
3 Blattfedersystem
4 Griffflächen
5 Spiralfeder
6 Kolben
7 erste Verriegelung
8 Verriegelungsnocken
9 zweite Verriegelung
10 weiterer Verriegelungsnocken
11 Federelemente
12 Federführung
13 Einstichanschlag
14 Faltenbalg
15 Schutzkappe
16 Injektionsnadel
17 Spritzenkörper
18 Stopfen
19 Einrastflansch
20 Ausbringelement
21 Ausbringhülse
22 Blattfederboden

## Patentansprüche

1. Spritze mit einem Ausbringelement (20) und einem Lagerelement (2), wobei das Ausbringelement (20) eine Ausbringhülse (21) und ein Blattfedersystem (3) aufweist,
wobei das Lagerelement (2) einen Spritzenkörper (17) aufweist, und einends an dem Spritzenkörper (17) eine Injektionsnadel (16) und andernends ein Einrastflansch (19) ausgebildet ist, wobei in dem Spritzenkörper (17) ein bewegbarer Stopfen (18) angeordnet ist,
wobei das Blattfedersystem (3) einen Kolben (6) und eine um den Kolben (6) herum angeordnete Spiralfeder (5) umfasst, wobei der Kolben (6) und die Spiralfeder (5) in die Ausbringhülse (21) einschiebbar ist und die Spiralfeder (5) zwischen einem Blattfederboden (22) angeordnet ist, wobei der Kolben (6) an den Stopfen (18) anschlägt und die Spiralfeder (5) an einen Spiralfederanschlag des Lagerelements (2),
**dadurch gekennzeichnet,**
**dass** die Ausbringhülse (21) einen Führungskanal aufweist und die Ausbringhülse (21) eine erste Verriegelung (7) und eine zweite Verriegelung (9) umfasst, wobei der Einrastflansch (19) in dem Führungskanal bewegbar ist, und an der ersten Verriegelung (7) oder der zweiten Verriegelung (9) festlegbar und lösbar ist, wobei das Blattfedersystem (3) aus dem Blattfederboden (22) und einem Deckenbereich besteht, wobei der Deckenbereich die Ausbringhülse (21) umfasst, wobei der Kolben (6) von dem Blattfederboden (22) hin zum Deckenbereich erstreckt und zwischen dem Blattfederboden (22) und dem Deckenbereich ein Kompressionsfederelement einstückig mit dem Blattfederboden (22) und dem Deckenbereich ausgebildet ist, wobei das Kompressionsfederelement ein Rückhalteelement umfasst,
und die erste Verriegelung (7) den Einrastflansch (19) verriegelt und löst und die zweite Verriegelung (9) den weiteren Eintrastflansch verriegelt und löst, wobei die erste Verriegelung (7) der Verriegelung und der Festlegung des Lagerelements (2) in dem Ausbringelement (20) in einer ersten Position dient, wobei der Einrastflansch (19) des Lagerelements (2) in dem Führungskanal derart entlang gleitet bis es selbständig durch das erste Verriegelungselement (7) in der ersten Position verriegelt ist und
nach der manuellen Entriegelung der ersten Verriegelung (7) gleitet das Lagerelement (2) in der Ausbringhülse (20) proximal hin zu dem zu behandelnden Körper und wird von dem zweiten Verriegelungselement (9) festgelegt.

2. Spritze nach Anspruch 1, **dadurch gekennzeichnet, dass** die Ausbringhülse (21) einen weiteren Führungskanal aufweist, wobei ein weiterer Einrastflansch mit dem weiteren Führungskanal wirkverbindbar ist.

3. Spritze nach einem der vorigen Ansprüche, **dadurch gekennzeichnet, dass** dass der Stopfen (18) in dem Lagerelement (2) verschiebbar und abgedichtet ist.

4. Spritze nach einem der vorigen Ansprüche, **dadurch gekennzeichnet, dass** die erste Verriegelung (7) ein erstes Federelement umfasst, wobei das erste Federelement einstückig mit dem Blattfedersystem (3) verbunden ist.

5. Spritze nach einem der vorigen Ansprüche, **dadurch gekennzeichnet, dass** die zweite Verriegelung (19) ein zweites Federelement umfast, wobei das zweite Federelement einstückig mit dem Blattfedersystem (3) verbunden ist.

6. Spritze nach einem der vorigen Ansprüche, **dadurch gekennzeichnet, dass** die Ausbringhülse (21) eine Einstichanschlagsfläche (13) aufweist und andernends die erste Verriegelung (7) ausgebildet ist, wobei die Einstichanschlagsfläche (13) eine Aufnahme umfasst.

7. Spritze nach Anspruch 6, **dadurch gekennzeichnet, dass** die Einstichanschlagsfläche (13) einen Faltenbalg (14) umfasst.

8. Spritze nach Anspruch 7, **dadurch gekennzeichnet, dass** der Faltenbalg (14) in der Aufnahme angeordnet ist.

## Claims

1. Syringe having a discharge element (20) and a storage element (2), the discharge element (20) having a discharge sleeve (21) and a leaf spring system (3),
Wherein the storage element (2) has a syringe body (17), and an injection needle (16) is formed at one end on the syringe body (17) and an engagement flange (19) is formed at the other end, wherein a movable stopper (18) is arranged in the syringe body (17),
wherein the leaf spring system (3) comprises a plunger (6) and a spiral spring (5) arranged around the plunger (6), wherein the plunger (6) and the spiral spring (5) can be pushed into the discharge sleeve (21) and the spiral spring (5) is arranged between a leaf spring base (22), the plunger (6) abuts against the stopper (18) and the spiral spring (5) abuts against a spiral spring stop of the storage element (2),
**characterized in that**
the discharge sleeve (21) has a guide channel and the discharge sleeve (21) comprises a first locking means (7) and a second locking means (9), wherein the engagement flange (19) is movable in the guide channel and to is fixable to and releasable from the first locking means (7) or the second locking means (9), wherein the leaf spring system (3) is composed of the leaf spring base (22) and a ceiling region, said ceiling region comprises the discharge sleeve (21), wherein the plunger (6) extends from said leaf spring base (22) to the ceiling region, and between the leaf spring base (229 and the ceiling region, a compression spring element is integrally formed with the leaf spring base (22) and said ceiling region, wherein the compression spring element comprises a retention element,
and the first locking means (7) locks and releases the engagement flange (19) and the second locking means (9) locks and releases the further engagement flange,
the first locking means (7) serves to lock and secure the storage element (2) in the discharge element (20) in a first position, wherein the engagement flange (19) of the storage element (2) slides along in the guide channel in such a way that it is automatically locked by the first lock means (7) in the first position, and after manually unlocking of the first locking means (7), the storage element (2) slides proximal in the discharge sleeve (20) towards the body to be treated and is fixed by the second locking means (9).

2. Syringe according to claim 1, **characterized in that** the discharge sleeve (21) has a further guide channel, wherein a further engagement flange can be operatively connected to the further guide channel.

3. Syringe according to one of the previous claims, **characterized in that** the stopper (18) is displaceable and sealed in the storage element (2).

4. Syringe according to one of the previous claims, **characterized in that** the first locking means (7) comprises a first spring element, wherein the first spring element is integrally connected to the leaf spring system (3).

5. Syringe according to one of the previous claims, **characterized in that** the second locking means (9) comprises a second spring element, wherein the second spring element is integrally connected to the leaf spring system (3).

6. Syringe according to one of the previous claims, **characterized in that** the discharge sleeve (21) has a puncture abutment face (13) and the first locking means (7) is formed at the other end, wherein the puncture abutment face (13) comprises a receptacle.

7. Syringe according to claim 6, **characterized in that** the puncture abutment face (13) comprises a bellows (14).

8. Syringe according to claim 7, **characterized in that** the bellows (14) is arranged in the receptacle.

## Revendications

1. Seringue avec un élément de distribution (20) et un élément d'appui (2), dans laquelle l'élément de distribution (20) présente un manchon de distribution (21) et un système de ressort à lames (3),
dans lequel l'élément d'appui (2) présente un corps de seringue (17), et à une extrémité du corps de seringue (17) est réalisée une aiguille d'injection (16) et à l'autre extrémité est réalisée une bride d'emboîtement (19), dans lequel dans le corps de seringue (17) est disposé un obturateur mobile (18),
dans lequel le système de ressort à lames (3) comporte un piston (6) et un ressort hélicoïdal (5) disposé autour du piston (6), dans lequel le piston (6) et le ressort hélicoïdal (5) peuvent être introduits dans le manchon de distribution (21) et le ressort hélicoïdal (5) est disposé entre un fond de ressort à lame (22), dans lequel le piston (6) vient en butée contre l'obturateur (18) et le ressort hélicoïdal (5) vient en butée contre une butée de ressort hélicoïdal de l'élément d'appui (2),
**caractérisée par le fait que**
le manchon de distribution (21) présente un canal de guidage et le manchon de distribution (21) comporte un premier mécanisme de verrouillage (7) et un deuxième mécanisme de verrouillage (9), où la bride d'emboîtement (19) est déplaçable dans le canal de guidage et peut être fixée au et libérée du premier mécanisme de verrouillage (7) ou du deuxième mécanisme de verrouillage (9), où le système de ressort à lame (3) est constitué du fond de ressort à lame (22) et d'une zone de plafond, où la zone de plafond comporte le manchon de distribution (21), où le piston (6) s'étend du fond de ressort à lame (22) à la zone de plafond et, entre le fond de ressort à lame (22) et la zone de plafond, un élément de ressort de compression est formé d'une seule pièce avec le fond de ressort à lame (22) et la zone de plafond, où l'élément de ressort de compression comporte un élément de retenue,
et le premier mécanisme de verrouillage (7) verrouille et libère la bride d'emboîtement (19) et le deuxième mécanisme de verrouillage (9) verrouille et libère l'autre bride d'emboîtement,
dans lequel le premier mécanisme de verrouillage (7) sert à verrouiller et à fixer l'élément d'appui (2) dans l'élément de distribution (20) dans une première position, dans lequel la bride d'emboîtement (19) de l'élément d'appui (2) coulisse dans le canal de guidage jusqu'à ce qu'elle soit verrouillée de manière autonome par le premier élément de verrouillage (7) dans la première position, et
après le déverrouillage manuel du premier mécanisme de verrouillage (7), l'élément d'appui (2) coulisse dans le manchon de distribution (20) de manière proximale vers le corps à traiter et est fixé par le deuxième élément de verrouillage (9).

2. Seringue selon la revendication 1, **caractérisée par le fait que** le manchon de distribution (21) présente un autre canal de guidage, dans lequel une autre bride d'emboîtement peut être connectée de manière opérationnelle à l'autre canal de guidage.

3. Seringue selon l'une des revendications précédentes, **caractérisée par le fait que** l'obturateur (18) dans l'élément d'appui (2) est déplaçable et est rendu hermétique.

4. Seringue selon l'une des revendications précédentes, **caractérisée par le fait que** le premier mécanisme de verrouillage (7) comporte un premier élément de ressort, dans lequel le premier élément de ressort est connecté de manière à former une seule pièce avec le système de ressort à lame (3).

5. Seringue selon l'une des revendications précédentes, **caractérisée par le fait que** le deuxième mécanisme de verrouillage (9) comporte un deuxième élément de ressort, dans lequel le deuxième élément de ressort est connecté de manière à former une seule pièce avec le système de ressort à lame (3).

6. Seringue selon l'une des revendications précédentes, **caractérisée par le fait que** le manchon de distribution (21) présente une surface de butée de ponction (13) et qu'à l'autre extrémité est formé le premier mécanisme de verrouillage (7), dans lequel la surface de butée de ponction (13) comporte un moyen de réception.

7. Seringue selon la revendication 6, **caractérisée par le fait que** la surface de butée de ponction (13) comporte un soufflet (14).

8. Seringue selon la revendication 7, **caractérisée par le fait que** le soufflet (14) est disposé dans le moyen de réception.
